# EUROPEAN PATENT SPECIFICATION

(11) **EP 2 777 479 B1**
(45) Date of publication and mention of the grant of the patent: **20.05.2020**
(21) Application number: 14159038.0
(22) Date of filing: 12.03.2014
(51) Int. Cl.: A61B 1/00, A61M 25/01, A61M 25/00

(54) **Improved catheter stiffness adjustment system**
Verbessertes Kathetersteifheitseinstellungssystem
Système de réglage amélioré de rigidité de cathéter

(30) Priority: 13.03.2013 US 201313799821
(43) Date of publication of application: 17.09.2014
(73) Proprietor: DePuy Synthes Products, LLC, Raynham, MA 02767 (US)
(72) Inventor: Arguello, Edward, Weston, FL Florida 33331 (US)
(74) Representative: Carpmaels & Ransford LLP

(56) References cited:
- EP-A1- 1 709 987
- WO-A2-2007/028058
- US-A1- 2007 112 331
- US-A1- 2011 230 718

## Description

### BACKGROUND OF THE INVENTION

### 1. Field of the Invention

The invention relates to catheters insertable within vasculature of a patient and more particularly to mechanisms and systems that alter the stiffness of at least one section of the catheter.

### 2. Description of the Related Art

Traditional vascular catheter designs struggle with the trade-off between flexibility and stiffness. Flexibility is desired to assist in navigating tortuous anatomy of the vasculature. On the other hand, stiffness is needed both for pushability during insertion and, after the distal tip of the catheter is positioned at a selected site, for stability to provide support during the advancement of accessory devices through the catheter.

There are a number of systems for steering catheters, such as disclosed by Lundquist et al. in U.S. Patent No. 5,254,088 and by Heinzelman et al. in U.S. Patent No. 5,364,351. Steering mechanisms described in these patents have levers or knobs which cause selective rotation or tension on steering wires or other elements within the catheters.

Several constructions of an adjustable stiffness catheter are described by Gregorich et al. in U.S. Patent Publication No. 2007/0060880. In one construction having coaxial hypotubes, the inner diameter of an outer hypotube is decreased, or the outer diameter of an inner hypotube is increased, to engage one hypotube with the other hypotube. In other constructions, one or more inflatable elements engage a hypotube.

It is therefore desirable to have an improved catheter stiffness adjustment mechanism that is simple to use and which does not bind inner and outer elements together.

US2011/0230718A1 discloses a medical flexible section that includes an inner tube body which has a first bend allowing section configured to allow bending in a predetermined direction provided on a side surface thereof and forms a duct and an outer tube body which has a second bend allowing section configured to allow bending in a predetermined direction provided on a side surface thereof and in which the inner tube body is inserted. The medical flexible section includes an operation ring configured to change bendability of an insertion section constituted of the outer tube body and the inner tube body by rotating the inner tube body in a periaxial direction with respect to the outer tube body to adjust a relative positional relationship between the first bend allowing section and the second bend allowing section.

### SUMMARY OF THE INVENTION

An object of the present invention is to provide an improved stiffness adjustment system that can be actuated as desired during insertion and placement of a catheter to change its flexibility.

The present invention is defined in claim 1. Preferred embodiments of the invention are defined in the dependent claims.

### BRIEF DESCRIPTION OF THE DRAWINGS

In what follows, preferred embodiments of the invention are explained in more detail with reference to the drawings, in which:
FIG. 1 is a schematic cross-sectional view through a region of a catheter according to the present invention having an outer catheter body containing first and second members movable relative to each other;
FIG. 2 is a diagrammatic side view of first and second members aligned in phase to generate a first flexure condition;
FIG. 3 is a diagrammatic side view of first and second members aligned out of phase to generate a second, stiffer flexure condition;
FIG. 4 is a schematic side view of first and second members separated from each other for illustrative purposes and aligned in phase to generate a first flexure condition when positioned concentrically;
FIG. 5 is a schematic similar to FIG. 4 with the first and second members aligned out of phase to generate a second, stiffer flexure condition when concentric;
FIG. 6 is a schematic perspective view of a proximal portion of the catheter of FIG. 1 having an actuation mechanism to move the first and second members relative to each other;; and
FIG. 7 is a schematic side view of a distal portion of first and second actuation members.

### DETAILED DESCRIPTION OF THE PRESENTLY PREFERRED EMBODIMENTS

This invention may be accomplished by a vascular catheter having adjustable stiffness during insertion into vasculature of a patient, where the terms "vascular" and "vasculature" are utilized in their broadest meaning to include any duct or tube network in a human or other animal. A catheter according to the present invention includes a catheter stiffener adjustment system having a first member with an outer diameter and having at least three first segments with at least one joint enabling the segments to bend relative to each other, and a second member having an inner diameter defining a passageway through which the first member is movable relative to the second member. The second member has at least three second segments with at least one joint enabling the segments to bend relative to each other. In a first position, the first segments and the second segments are alignable substantially in phase to generate a first flexure condition, also referred to as a "flexibility mode". In a second position, the first segments and the second segments are alignable substantially out of phase to generate a second, stiffer flexure condition, also referred to as a "stiffness mode".

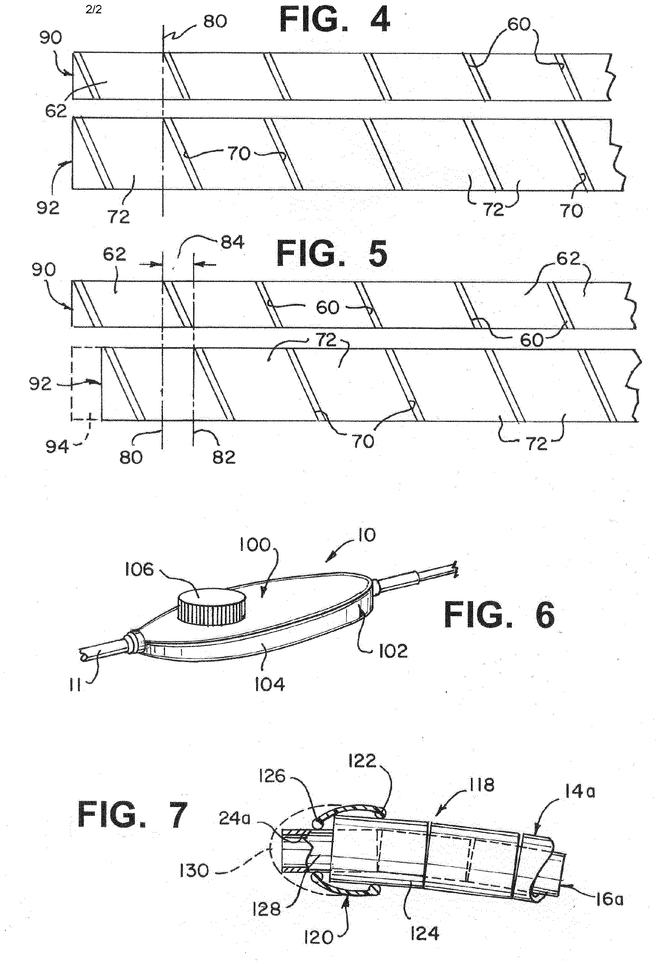

The present invention solves the trade-off faced by most conventional catheters by providing a system that generates an amount of stiffness that can be changed as desired, prior to or during a surgical procedure, by utilizing a simple actuation mechanism such as described below in relation to FIG. 6. Therefore, a surgeon or other clinician can activate the stiffness mode for pushability, including accepting a catheter already placed in stiffness mode by a manufacturer, advance the catheter into vasculature utilizing the stiffness mode, and then transition to the flexible mode to navigate tortuous distal anatomy. The clinician can finally return to the stiffness mode once the working portion of the catheter, typically at or near the distal end of the catheter, has reached the target anatomy selected by the clinician to provide stability and support during the delivery of accessory devices. In addition, the adjustable stiffness system is segmentable for different lengths, allowing for particular sections or regions of a catheter to alternate between flexibility and stiffness modes independently.

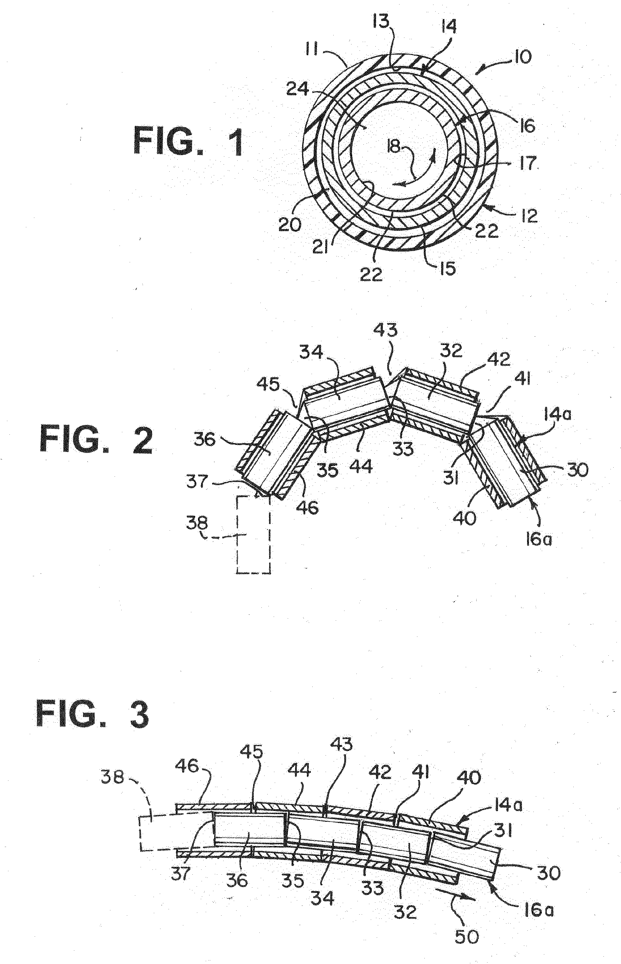

A catheter 10, shown in cross-section in FIG. 1 as one construction according to the present invention, includes an outer catheter body 12, typically formed of a biocompatible polymeric material, having an outer surface 11 and an inner surface 13 defining a central lumen 20. A second, outer member 14 has an outer surface 15 and an inner surface 17 defining a passageway 22. A first, inner member 16 is movably disposed within passageway 22 and has an outer surface 19 and an inner surface 21 defining a passageway 24. The first and second members 14, 16, formed of metal or polymeric hypotubes in some constructions, are movable relative to each other, such as by rotation indicated by arrow 18.

FIG. 2 is a diagrammatic side view of first and second members 16a, 14a aligned in phase to generate a first flexure condition, also referred to as a flexibility mode. In this construction, first member 16a includes segments 30, 32, 34 and 36 separated by joints 31, 33 and 35. Dashed line 38 represents additional segments in some constructions, with at least one further joint 37, and represents non-segmented regions in other constructions. Similarly, second member 14a has segments 40, 42, 44 and 46 separated by joints 41, 43 and 45 spaced from each other at substantially the same frequency or periodicity as for joints 31, 33 and 35 of the first member 16a.

FIG. 3 is a diagrammatic side view of first and second members 16a, 14a aligned out of phase to generate a second, stiffer flexure condition also referred to as a stiffness mode. In this construction, first member 16a has been retracted in a proximal direction, toward a clinician operating the alignment system, as indicated by arrow 50.

FIG. 4 is a schematic side view of first and second members 14b, 16b separated from each other for illustrative purposes and aligned in phase to generate a first flexure condition when positioned concentrically as shown in FIG. 1. In this construction, a single helical joint 60 separates segments 62 of first member 16b while single helical joint 70 separates segments 72 of second member 14b. Joints 60 and 70 are aligned in phase as indicated by dashed line 80.

FIG. 5 is a schematic similar to FIG. 4 with the first and second members 16b, 14b aligned out of phase to generate a second, stiffer flexure condition when concentric. In this construction, second member 14b has been retracted proximally, arrow 78, to move joint 70 by a distance 84 to a new position represented by dashed line 82. Distance 84 is less than the spacing between segments as indicated by arrow 86. Solid ends 90 and 92 represent relative positions when first and second members 16a, 14a are translated relative to each other; dashed line 94 represents the unchanged longitudinal position of second member 14b if the first and second members are simply rotated relative to each other to achieve the out-of-phase alignment shown in FIG. 5.

In other words, this invention may be accomplished by a two-member system, each member having one or more joints of substantially the same frequency or periodicity as the other member to thereby establish segments having similar lengths, preferably substantially identical lengths, and enable the segments of the first and second members to be controllably aligned in phase or out of phase, depending on the desired amount of flexibility or stiffness for the members and accompanying structure such as an outer catheter body. In some constructions, the joints are formed by cuts, either continuous or discrete, which extend through the entire wall thickness of the member and, in other constructions, permeate to a selected wall thickness to create living hinges. One suitable source for metal hypotubes, formed of a platinum alloy or tantalum if radiopacity is desired, is Johnson Matthey Medical Components (see "www.jmmedical.com"). If metal or polymeric hypotubes or other hollow, substantially cylindrical tubes are utilized, it is generally easier to manufacture the joints as cuts made by laser or water jet into the outer diameters of the tubes. The segments then have a major or outer diameter, with a maximum wall thickness, while the joints have a minor or smaller diameter with minimal or no wall thickness, depending on the depth of the cuts. Partial cuts with minimal wall thickness can also be referred to as channels or grooves between segments.

Additionally, the joints, as continuous or discrete bending loci, can be formed on all of the length of the tubes or other elongated items serving as first and second members, or can be formed only on certain sections of the first and second members. Further, each member may also vary in thickness, material and/or durometer to generate a desired amount of flexibility or stiffness over selected lengths for the respective modes to tailor the stiffener adjustment system to particular procedures. Moreover, the periodic cut frequency, cut type and shape, and material of the two members can be adjusted to manage catheter kink radius, mean and differential stiffness between the two modes or states of flexibility, and length change needed to transition between the two modes or states.

FIG. 6 is a schematic perspective view of a proximal portion 100 of catheter 10, FIG. 1, having an actuation mechanism 102 with a housing 104 and control knob 106 to move the first and second members relative to each other, such as by a sliding or twisting motion to translate and/or rotate one or both members. In other constructions, a control lever or toggle is provided, which is purely mechanical in some forms and electro-mechanical in other forms.

FIG. 7 is a schematic side view of a distal portion 118 of first and second actuation members 16a, 14a of FIG. 2 with an elastomeric cover 120 that enables relative motion between the first and second members. In this construction, cover 120 has a first O-ring-type seal 122 contacting outer segment 124 of second member 14a and has a second O-ring-type seal 126 contacting inner segment 128 of first member 16a. Accessory devices can be advanced distally through a passageway 24a in first member 16a.

Dashed line 130 represents another construction in which cover 120 has a dome-like distal end which occludes the distal opening of passageway 24a. Cover 120 is sealed entirely by seal 122 on outer segment 124, obviating the need for seal 126.

Biocompatible jacketing materials, inner liners, outer liners, and/or coatings can be applied to or over inner and/or outer surfaces of one or both of the first and second members as desired for particular procedures and to enhance compatibility with devices or fluids passed through catheters with adjustable stiffness according to the present invention. For example, imaging agents, pharmaceuticals, saline or other liquids may be injected under pressure. Liners or spacers can assist alignment of the joints of both members during use, and preferably reduce frictional engagement between the members within tortuous vascular pathways.

A method of adjusting the stiffness of a catheter includes selecting a catheter having an outer catheter body defining a central lumen, a first member with an outer diameter and having at least three first segments with at least one joint enabling the segments to bend relative to each other, and a second member movable through the central lumen of the catheter body and having an inner diameter defining a passageway through which the first member is movable relative to the second member. The second member has at least three second segments with at least one joint enabling the segments to bend relative to each other. At least the three first segments and the three second segments have sufficiently similar lengths to enable alignment substantially in phase with the first and second members in a first position and, in a second position, being alignable substantially out of phase. The method further includes aligning the first segments and the second segments substantially in phase to generate a first flexure condition for the catheter. The method also includes aligning the first segments and the second segments substantially out of phase to generate a second, stiffer flexure condition for the catheter.

Thus, while there have been shown, described, and pointed out fundamental novel features of the invention as applied to a preferred embodiment thereof, it will be understood that various omissions, substitutions, and changes in the form and details of the devices illustrated, and in their operation, may be made by those skilled in the art without departing from the scope of the invention, which is defined by the appended claims. It is also to be understod that the drawing are not necessarily drawn to scale, but that they are merely conceptual in nature.

## Claims

1. A catheter stiffener adjustment system, comprising:
a first member (16a) having an outer diameter and having at least three first segments (30, 32, 34, 36) with at least one joint (31, 33, 35) enabling the segments (30, 32, 34, 36) to bend relative to each other;
a second member (14a) having an inner diameter defining a passageway through which the first member (16a) is movable relative to the second member (14a), and having at least three second segments (40, 42, 44, 46) with at least one joint (41, 43, 45) enabling the segments (40, 42, 44, 46) to bend relative to each other;
in a first position, the first segments (30, 32, 34, 36) and the second segments (40, 42, 44, 46) are alignable substantially in phase to generate a first flexure condition; and
in a second position, the first segments (30, 32, 34, 36) and the second segments (40, 42, 44, 46) are alignable substantially out of phase to generate a second, stiffer flexure condition
wherein the first member (16a) and the second member (14a) are translatable relative to each other between both the first and second positions.

2. The system of claim 1 wherein the first member (16a) and the second member (14a) are at least one of rotatable and translatable relative to each other in both the first and second positions.

3. The system of claim 1 wherein the joint (31, 33, 35, 41, 43, 45) for at least one of the first and second members (16a, 14a) is substantially helical.

4. The system of claim 1 wherein the joint (31, 33, 35, 41, 43, 45) for at least one of the first and second members (16a, 14a) is formed by a thinning of material separating the segments (30, 32, 34, 36, 40, 42, 44, 46).

5. The system of claim 1 wherein the joint (31, 33, 35, 41, 43, 45) for at least one of the first and second members (16a, 14a) is formed by an elimination of material separating the segments (30, 32, 34, 36, 40, 42, 44, 46) along at least one side of the member (16a, 14a).

6. The system of claim 1 wherein each of the first and second members (16a, 14a) is substantially cylindrical.

7. The system of claim 6 wherein the inner diameter of the second member (14a) is greater than the outer diameter of the first member (16a) in both the first and second positions.

8. The system of claim 1, where:
the first member (16a) is substantially tubular;
the second member (14a) is substantially tubular and is disposed substantially concentrically about the first member (16a) and wherein the first member (16a) is movable relative to the second member (14a) by at least one of rotation and translation, the inner diameter of the second member (14a) being greater than the outer diameter of the first member (16a), and wherein at least the three first segments (30, 32, 34, 36) and the three second segments (40, 42, 44, 46) have sufficiently similar lengths to enable alignment substantially in phase with the first and second members (16a, 14a) in the first position and, in the second position, being alignable substantially out of phase; and
in the second position, the first member (16a) and the second member (14a) being at least one of rotatable and translatable relative to each other in both the first and second positions.

9. The system of claim 8 wherein the joint (31, 33, 35, 41, 43, 45) for each of the first and second members (16a, 14a) is substantially helical and substantially continuous.

10. A catheter (10) having a stiffener adjustment system of claim 1, comprising:
an outer catheter body (12) having a central lumen (20); and
wherein;
the second member (14a) is movable through the central lumen (20) of the catheter body (12);
in the first position, the first segments (30, 32, 34, 36) and the second segments (40, 42, 44, 46) are alignable substantially in phase to generate the first flexure condition for the catheter (10); and
in the second position, the first segments (30, 32, 34, 36) and the second segments (40, 42, 44, 46) are alignable substantially out of phase to generate the second, stiffer flexure condition for the catheter (10).

11. The catheter (10) of claim 10 wherein the first member (15a) and the second member (14a) are at least one of rotatable and translatable relative to each other in both the first and second positions by moving an actuation mechanism (102) connected to at least one of the first and second members (16a, 14a).

12. The catheter (10) of claim 11 wherein the joint (40, 42, 44, 46) for at least one of the first and second members (16a, 14a) is substantially helical and substantially continuous.

13. The system of claim 1 wherein at least one of the first and second members (16a, 14a) is substantially tubular.

14. The catheter (10) of claim 10 wherein the catheter body has a plurality of longitudinal sections, with segments underlying at least one section to be capable of controlling flexibility for that section.

## Patentansprüche

1. Katheterversteifungselementverstellsystem, umfassend:
ein erstes Glied (16a), das einen äußeren Durchmesser und mindestens drei erste Segmente (30, 32, 34, 36) mit mindestens einem Gelenk (31, 33, 35) hat, das es den Segmenten (30, 32, 34, 36) ermöglicht, sich bezüglich einander zu biegen,
ein zweites Glied (14a), das einen inneren Durchmesser, der einen Durchgang definiert, durch den das erste Glied (16a) bezüglich des zweiten Glieds (14a) beweglich ist, und mindestens drei zweite Segmente (40, 42, 44, 46) mit mindestens einem Gelenk (41, 43, 45) hat, das es den Segmenten (40, 42, 44, 46) ermöglicht, sich bezüglich einander zu biegen,
wobei die ersten Segmente (30, 32, 34, 36) und die zweiten Segmente (40, 42, 44, 46) in einer ersten Position im Wesentlichen in Phase aufeinander ausrichtbar sind, um einen ersten Biegungszustand zu erzeugen, und
wobei die ersten Segmente (30, 32, 34, 36) und die zweiten Segmente (40, 42, 44, 46) in einer zweiten Position im Wesentlichen außer Phase aufeinander ausrichtbar sind, um einen zweiten, steiferen Biegungszustand zu erzeugen,
wobei das erste Glied (16a) und das zweite Glied (14a) zwischen sowohl der ersten als auch der zweiten Position bezüglich einander translatierbar sind.

2. System nach Anspruch 1, wobei das erste Glied (16a) und das zweite Glied (14a) sowohl in der ersten als auch in der zweiten Position bezüglich einander drehbar sind.

3. System nach Anspruch 1, wobei das Gelenk (31, 33, 35, 41, 43, 45) für das erste und/oder das zweite Glied (16a, 14a) im Wesentlichen schraubenförmig ist.

4. System nach Anspruch 1, wobei das Gelenk (31, 33, 35, 41, 43, 45) für das erste und/oder das zweite Glied (16a, 14a) durch ein Verdünnen von Material, das die Segmente (30, 32, 34, 36, 40, 42, 44, 46) trennt, gebildet ist.

5. System nach Anspruch 1, wobei das Gelenk (31, 33, 35, 41, 43, 45) für das erste und/oder das zweite Glied (16a, 14a) durch ein Entfernen von Material, das die Segmente (30, 32, 34, 36, 40, 42, 44, 46) trennt, entlang mindestens einer Seite des Glieds (16, 14a) gebildet ist.

6. System nach Anspruch 1, wobei das erste und das zweite Glied (16a, 14a) jeweils im Wesentlichen zylindrisch ist.

7. System nach Anspruch 6, wobei der innere Durchmesser des zweiten Glieds (14a) sowohl in der ersten als auch in der zweiten Position größer als der äußere Durchmesser des ersten Glieds (16a) ist.

8. System nach Anspruch 1, wobei:
das erste Glied (16a) im Wesentlichen röhrenförmig ist,
das zweite Glied (14a) im Wesentlichen röhrenförmig und im Wesentlichen konzentrisch um das erste Glied (16a) angeordnet ist und wobei das erste Glied (16a) durch Translation oder Translation und Drehung bezüglich des zweiten Glieds (14a) beweglich ist, wobei der innere Durchmesser des zweiten Glieds (14a) größer als der äußere Durchmesser des ersten Glieds (16a) ist und wobei mindestens die drei ersten Segmente (30, 32, 34, 36) und die drei zweiten Segmente (40, 42, 44, 46) hinreichend ähnliche Längen haben, so dass sie in der ersten Position auf das erste und zweite Glied (16a, 14a) im Wesentlichen in Phase ausgerichtet werden können und in der zweiten Position im Wesentlichen außer Phase ausrichtbar sind, und
das erste Glied (16a) und das zweite Glied (14a) in der zweiten Position sowohl in der ersten als auch in der zweiten Position bezüglich einander translatierbar und drehbar sind.

9. System nach Anspruch 8, wobei das Gelenk (31, 33, 35, 41, 43, 45) für jeweils das erste und das zweite Glied (16a, 14a) im Wesentlichen schraubenförmig und im Wesentlichen durchgängig ist.

10. Katheter (10) mit einem Versteifungselementverstellsystem nach Anspruch 1, umfassend:
einen äußeren Katheterkörper (12) mit einem mittleren Lumen (20) und
wobei
das zweite Glied (14a) durch das mittlere Lumen (20) des Katheterkörpers (12) beweglich ist,
die ersten Segmente (30, 32, 34, 36) und die zweiten Segmente (40, 42, 44, 46) in der ersten Position im Wesentlichen in Phase aufeinander ausrichtbar sind, um den ersten Biegungszustand für den Katheter (10) zu erzeugen, und
die ersten Segmente (30, 32, 34, 36) und die zweiten Segmente (40, 42, 44, 46) in der zweiten Position im Wesentlichen außer Phase aufeinander ausrichtbar sind, um den zweiten, steiferen Biegungszustand für den Katheter (10) zu erzeugen.

11. Katheter (10) nach Anspruch 10, wobei das erste Glied (15a) und das zweite Glied (14a) sowohl in der ersten als auch in der zweiten Position durch Bewegen eines Betätigungsmechanismus (102), der mit dem ersten und/oder dem zweiten Glied (16a, 14a) verbunden ist, bezüglich einander drehbar und translatierbar sind.

12. Katheter (10) nach Anspruch 11, wobei das Gelenk (40, 42, 44, 46) für das erste und/oder das zweite Glied (16a, 14a) im Wesentlichen schraubenförmig und im Wesentlichen durchgängig ist.

13. System nach Anspruch 1, wobei das erste und/oder das zweite Glied (16a, 14a) im Wesentlichen röhrenförmig ist.

14. Katheter (10) nach Anspruch 10, wobei der Katheterkörper eine Vielzahl von länglichen Abschnitten hat, wobei Segmente, die unter mindestens einem Abschnitt liegen, in der Lage sein sollen, die Flexibilität für diesen Abschnitt zu steuern.

## Revendications

1. Système de réglage d'élément raidisseur de cathéter, comprenant :
un premier élément (16a) ayant un diamètre extérieur et comportant au moins trois premiers segments (30, 32, 34, 36) avec au moins une articulation (31, 33, 35) permettant aux segments (30, 32, 34, 36) de se courber les uns par rapport aux autres ;
un second élément (14a) ayant un diamètre intérieur définissant un passage à travers lequel le premier élément (16a) est mobile par rapport au second élément (14a), et ayant au moins trois seconds segments (40, 42, 44, 46) avec au moins une articulation (41, 43, 45) permettant aux segments (40, 42, 44, 46) de se courber les uns par rapport aux autres ;
dans une première position, les premiers segments (30, 32, 34, 36) et les seconds segments (40, 42, 44, 46) peuvent être alignés sensiblement en phase pour générer une première condition de flexion ; et
dans une seconde position, les premiers segments (30, 32, 34, 36) et les seconds segments (40, 42, 44, 46) peuvent être alignés sensiblement de manière déphasée pour générer une seconde condition de flexion plus rigide,
le premier élément (16a) et le second élément (14a) pouvant être déplacés l'un par rapport à l'autre entre la première et la seconde position.

2. Système selon la revendication 1 dans lequel le premier élément (16a) et le second élément (14a) peuvent tourner l'un par rapport à l'autre à la fois dans la première position et dans la seconde position.

3. Système selon la revendication 1 dans lequel l'articulation (31, 33, 35, 41, 43, 45) pour au moins l'un des premier et second éléments (16a, 14a) est sensiblement hélicoïdale.

4. Système selon la revendication 1 dans lequel l'articulation (31, 33, 35, 41, 43, 45) pour au moins l'un des premier et second éléments (16a, 14a) est formée par un amincissement du matériau séparant les segments (30, 32, 34, 36, 40, 42, 44, 46).

5. Système selon la revendication 1 dans lequel l'articulation (31, 33, 35, 41, 43, 45) pour au moins l'un des premier et second éléments (16a, 14a) est formée par une élimination de matériau séparant les segments (30, 32, 34, 36, 40, 42, 44, 46) le long d'au moins un côté de l'élément (16a, 14a).

6. Système selon la revendication 1 dans lequel chacun des premier et second éléments (16a, 14a) est sensiblement cylindrique.

7. Système selon la revendication 6, dans lequel le diamètre intérieur du second élément (14a) est supérieur au diamètre extérieur du premier élément (16a) à la fois dans la première position et dans la seconde position.

8. Système selon la revendication 1, dans lequel :
le premier élément (16a) est sensiblement tubulaire ;
le second élément (14a) est sensiblement tubulaire et est disposé de manière sensiblement concentrique autour du premier élément (16a) et le premier élément (16a) étant mobile par rapport au second élément (14a) par translation ou par translation et rotation, le diamètre intérieur du second élément (14a) étant supérieur au diamètre extérieur du premier élément (16a), et au moins les trois premiers segments (30, 32, 34, 36) et les trois seconds segments (40, 42, 44, 46) ayant des longueurs suffisamment similaires pour permettre un alignement sensiblement en phase avec les premier et second éléments (16a, 14a) dans la première position et, dans la seconde position, pouvant être alignés sensiblement de manière déphasée ; et
dans la seconde position, le premier élément (16a) et le second élément (14a) étant mobiles en translation et en rotation l'un par rapport à l'autre à la fois dans la première position et dans la seconde position.

9. Système selon la revendication 8 dans lequel l'articulation (31, 33, 35, 41, 43, 45) pour chacun des premier et second éléments (16a, 14a) est sensiblement hélicoïdale et sensiblement continue.

10. Cathéter (10) ayant un système d'ajustement d'élément raidisseur selon la revendication 1, comprenant :
un corps de cathéter extérieur (12) ayant une lumière centrale (20) ; et
dans lequel ;
le second élément (14a) est mobile à travers la lumière centrale (20) du corps de cathéter (12);
dans la première position, les premiers segments (30, 32, 34, 36) et les seconds segments (40, 42, 44, 46) peuvent être alignés sensiblement en phase pour générer la première condition de flexion pour le cathéter (10) ; et
dans la seconde position, les premiers segments (30, 32, 34, 36) et les seconds segments (40, 42, 44, 46) peuvent être alignés sensiblement de manière déphasée pour générer la seconde condition de flexion plus rigide pour le cathéter (10).

11. Cathéter (10) selon la revendication 10, dans lequel le premier élément (15a) et le second élément (14a) sont mobiles en rotation et en translation l'un par rapport à l'autre à la fois dans la première position et dans la seconde position en déplaçant un mécanisme d'actionnement (102) relié à au moins l'un des premier et second éléments (16a, 14a).

12. Cathéter (10) selon la revendication 11, dans lequel l'articulation (40, 42, 44, 46) d'au moins l'un des premier et second éléments (16a, 14a) est sensiblement hélicoïdale et sensiblement continue.

13. Système selon la revendication 1 dans lequel au moins l'un des premier et second éléments (16a, 14a) est sensiblement tubulaire.

14. Cathéter (10) selon la revendication 10, dans lequel le corps de cathéter présente une pluralité de sections longitudinales, avec des segments sous-jacents à au moins une section pour être capable de contrôler la flexibilité de cette section.
